# EUROPEAN PATENT APPLICATION

(11) **EP 4 032 513 A1**
(43) Date of publication of application: **27.07.2022**
(21) Application number: 20866861.6
(22) Date of filing: 03.09.2020
(51) Int. Cl.: A61F 5/56

(54) **MOUTHPIECE**

(30) Priority: 19.09.2019 JP 2019170696
(71) Applicant: Mitsui Chemicals, Inc., Tokyo 105-7122 (JP)
(72) Inventor: YUKITA, Takashi, Chiba 299-0265 (JP); IWABUCHI, Yuki, Chiba 299-0265 (JP); HASEGAWA, Akira, Chiba 299-0265 (JP)
(74) Representative: Epping - Hermann - Fischer
(86) International application number: PCT/JP2020/033377
(87) International publication number: WO 2021/054133

(57) **Abstract**

The present invention addresses the problem of providing a mouthpiece that has an excellent fit and that is not prone to breakage or deformation. This mouthpiece for solving the above problem has: a pair of mouthpiece body parts configured from an upper-jaw mouthpiece body part mounted to the upper jaw, and a lower-jaw mouthpiece body part mounted to the lower jaw; a positioning member for restricting rearward displacement of the lower-jaw mouthpiece body part; and a support part that is disposed between the positioning member and the body parts and that supports the positioning member, so as to cover a portion of a surface that faces a surface of the upper-jaw mouthpiece body part and/or the lower-jaw mouthpiece body part that is mounted to a tooth row, the support part including a material that has a different flexural modulus than the mouthpiece body parts.

## Description

### Technical Field

The present invention relates to an oral appliance.

### Background Art

It is said that obstructive sleep apnea syndrome is mainly caused with a lower jaw sagging and obstructing an airway. In consideration of the above point, various oral appliances have been proposed to remedy the obstructive sleep apnea syndrome. Those oral appliances are intended to cure apnea or snoring by restricting rearward displacement of the lower jaw of a user and keeping the airway wide.

For example, Patent Literature (hereinafter, abbreviated as PTL) 1 proposes an oral appliance including a pair of oral appliance body portions corresponding to an upper jaw and a lower jaw, and a metal-made coupling member coupling the oral appliance body portions to each other. According to the proposed oral appliance, the coupling member adjusts the relative positions of an upper-jaw oral appliance body portion and a lower-jaw oral appliance body portion and displaces the lower jaw of a user wearing the oral appliance forward. In the proposed oral appliance, the coupling member is fixed to the oral appliance body portions made of resin by using, for example, metal fittings.

Furthermore, PTL 2 discloses an oral appliance in which a pair of oral appliance body portions corresponding to an upper jaw and a lower jaw are connected to each other by using a flexible band. According to the oral appliance disclosed in PTL 2, the flexible band adjusts the relative positions of an upper-jaw oral appliance body portion and a lower-jaw oral appliance body portion and restricts rearward displacement of the lower jaw of a user. In the oral appliance disclosed in PTL 2, the flexible band is fixed to the oral appliance body portions made of a hard material, such as polycarbonate resin or acrylic resin, by using an adhesive.

### Citation List

### Patent Literatures

PTL 1
   U.S. Unexamined Patent Application Laid-Open No. 2007/0224567
PTL 2
   WO 2008/023799

### Summary of Invention

### Technical Problem

In each of the oral appliances disclosed in PTL 1 and PTL 2, the oral appliance body portions are made of materials with high hardness. The oral appliance body portions with high hardness tend to make the user wearing the oral appliance feel uncomfortable. On the other hand, if the oral appliance body portions are entirely made of soft materials to improve wearing feel, there may occur such a problem that the oral appliance body portions are damaged or deformed near the coupling member, and that the rearward displacement of the lower jaw is not sufficiently restricted.

The present invention has been accomplished in consideration of the above-described problem. Specifically, an object of the present invention is to provide an oral appliance that gives good wearing feel and that is less susceptible to damage and deformation.

### Solution to Problem

To solve the above-described problem, the present invention provides an oral appliance configured as follows.

The oral appliance includes a pair of oral appliance body portions constituted by an upper-jaw oral appliance body portion fitted to an upper jaw and a lower-jaw oral appliance body portion fitted to a lower jaw, a positioning member that restricts rearward displacement of the lower-jaw oral appliance body portion, and a support portion that is disposed between the positioning member and at least one of the oral appliance body portions to cover part of a surface of at least one of the upper-jaw oral appliance body portion and the lower-jaw oral appliance body portion, the surface being opposite to another surface fitted to a tooth row, and that supports the positioning member, wherein the support portion includes a material with a different flexural modulus from the oral appliance body portions.

### Advantageous Effects of Invention

According to the present invention, the oral appliance giving good wearing feel and being less susceptible to damage and deformation is provided.

### Brief Description of Drawings

FIG. 1 is a schematic front view of an oral appliance according to Embodiment 1 of the present invention;
FIG. 2Ais a schematic side view of the oral appliance, illustrated in FIG. 1, in a mouth closed state, and FIG. 2B is a schematic side view of the oral appliance, illustrated in FIG. 1, in a mouth open state;
FIG. 3A is a schematic perspective view illustrating an upper jaw side of an oral appliance according to Embodiment 2 of the present invention, and FIG. 3B is a schematic perspective view illustrating a lower jaw side of the oral appliance according to Embodiment 2 of the present invention;
FIG. 4 is a schematic side view of the oral appliance, illustrated in FIGS. 3A and 3B, in a mouth closed state;
FIG. 5 is a schematic perspective view of an oral appliance according to Embodiment 3 of the present invention;
FIG. 6 is a schematic side view of the oral appliance, illustrated in FIG. 5, in a mouth closed state;
FIG. 7 is a schematic perspective view of a positioning member of the oral appliance illustrated in FIG. 5; and
FIG. 8 is a schematic exploded perspective view of the positioning member illustrated in FIG. 7.

### Description of Embodiments

An oral appliance according to the present invention will be described below in connection with several embodiments. In the following description, the words "front" and "rear" indicate, respectively, a direction toward the front of a user wearing the oral appliance (namely, a direction toward a lip side within an oral cavity when viewed from a tongue body) and a direction toward the rear of the user wearing the oral appliance (namely, a direction toward a throat side within the oral cavity when viewed from the tongue body). The word "downward" indicates a direction toward a lower side of the user wearing the oral appliance (namely, a direction from the top of a head toward a foot when the user is upright). The word "left-right direction" indicates leftward and rightward directions with an exact middle of an upper jaw of the user wearing the oral appliance being a reference when viewing forward of the user (specifically, directions toward cheeks on both sides when viewed from the exact middle of the upper jaw). The words "outer side" and "inner side" indicate, respectively, a side closer to a body surface of the user wearing the oral appliance and a side away from the body surface.

Furthermore, in the following description, when a mouth of the user wearing the oral appliance is closed, this situation is referred to as a "mouth closed state", and when the mouth of the user wearing the oral appliance is opened, this situation is referred to as a "mouth open state". In the mouth closed state, an upper-jaw oral appliance and a lower-jaw oral appliance are arranged such that their surfaces facing each other are substantially parallel. In the mouth open state, because the lower-jaw oral appliance is moved along an arc-shaped path when the mouth is opened, the upper-jaw oral appliance and the lower-jaw oral appliance are arranged such that their surfaces facing each other are non-parallel with an angle of about 45° at maximum formed between both the surfaces.

### 1. Embodiment 1

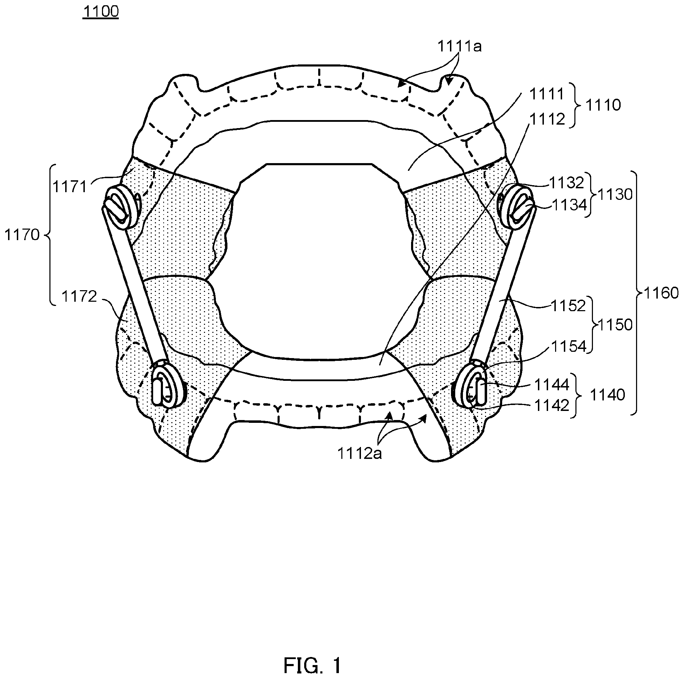

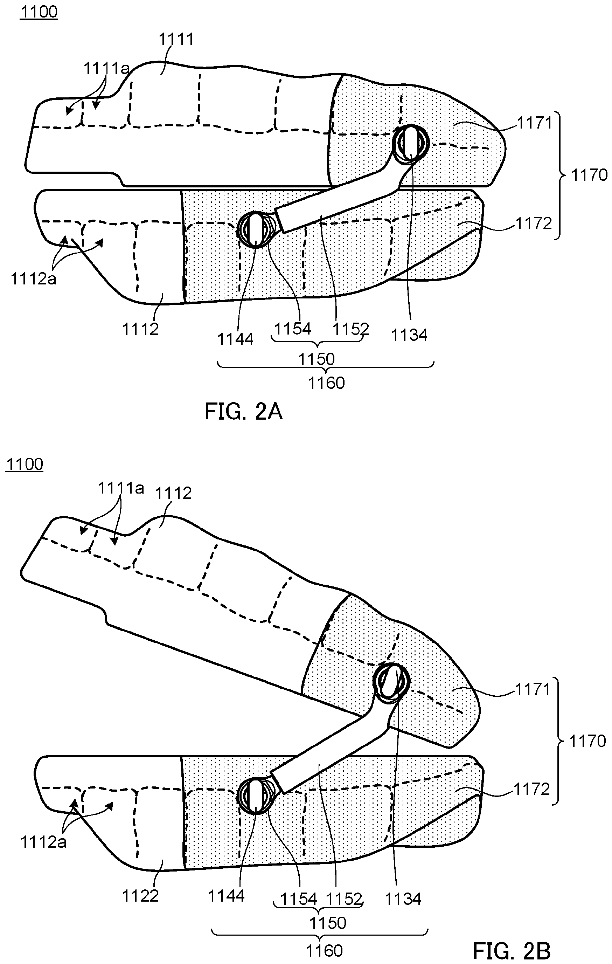

FIG. 1 is a schematic front view of oral appliance 1100 according to Embodiment 1 of the present invention, FIG. 2A is a schematic side view of oral appliance 1100 in the mouth closed state, and FIG. 2B is a schematic side view of oral appliance 1100n the mouth open state. As illustrated in FIGS. 1, 2A, and 2B, oral appliance 1100 according to this embodiment includes a pair of oral appliance body portions 1110 constituted by upper-jaw oral appliance body portion 1111 and lower-jaw oral appliance body portion 1112, a pair of left and right positioning members 1160 that restrict rearward displacement of lower-jaw oral appliance body portion 1112, and support portions 1170 constituted by a pair of left and right upper support portions 1171 that cover parts of upper-jaw oral appliance body portion 1111 and that support positioning members 1160, and by a pair of left and right lower support portions 1172 that cover parts of lower-jaw oral appliance body portion 1112 and that support positioning members 1160.

In oral appliance 1100 according to this embodiment, the positions of upper-jaw oral appliance body portion 1111 and lower-jaw oral appliance body portion 1112 are determined by positioning members 1160, and the position of a lower jaw relative to an upper jaw of the user is properly adjusted. As a result, during sleep of the user, an airway is kept wide, and apnea or snoring is cured.

In oral appliance 1100 according to this embodiment, the pair of oral appliance body portions 1110 and support portions 1170 have different flexural moduli. Furthermore, support portions 1170 are arranged to cover only parts of oral appliance body portions 1110. In this embodiment, for example, when the flexural modulus of a material contained in support portions 1170 is set to be higher than that of oral appliance body portions 1110, oral appliance body portions 1110 are easier to come into close contact with tooth rows of the user and are also deformable appropriately following the shapes of the tooth rows. This improves wearing feel of oral appliance 1100. On the other hand, support portions 1170 have a higher flexural modulus than oral appliance body portions 1110 and are less susceptible to deformation. Accordingly, positioning members 1160 can be supported at the desired position by support portions 1170, and support portions 1170 are less susceptible to damage and deformation even when load is applied to support portions 1170. As a result, oral appliance 1100 according to this embodiment can be stably used for a long period. In another case, the flexural modulus of the material contained in support portions 1170 may be set to be lower than that of oral appliance body portions 1110. This case can also improve the wearing feel by relieving force applied to oral appliance 1100 while damage and bending are suppressed.

Components of this embodiment will be described below.

### (Oral Appliance Body Portions)

As described above, oral appliance body portions 1110 is constituted by upper-jaw oral appliance body portion 1111 and lower-jaw oral appliance body portion 1112. Upper-jaw oral appliance body portion 1111 is fitted to the upper jaw of the user, and lower-jaw oral appliance body portion 1112 is fitted to the lower jaw of the user.

Each of oral appliance body portions 1110 includes a recess capable of receiving the tooth row. Although, in oral appliance body portions 1110 illustrated in FIGS. 1, 2A, and 2B, upper-jaw oral appliance body portion 1111 and lower-jaw oral appliance body portion 1112 include respectively tooth row mold portions 1111a and 1112a corresponding to the tooth rows of the user, the recesses are not always required to correspond to the shapes of the tooth rows. Oral appliance body portion 1110 may cover only part of the tooth row and is not always required to cover incisor teeth, for example. In such a case, structures covering left and right molar teeth are preferably coupled to each other by using, for example, a wire that is arranged along the tooth row. From the viewpoint of increasing strength of oral appliance body portion 1110 and improving the wearing feel, oral appliance body portion 1110 preferably has a structure covering the entire tooth row as illustrated in FIGS. 1, 2A, and 2B.

A thickness of each of oral appliance body portions 1110 is selected appropriately in accordance with the desired strength, the shape of the tooth row of the user, and so on. The thickness of oral appliance body portion 1110 may be substantially uniform in its entirety or may be changed as required. For example, a region of oral appliance body portion 1110 covered with support portion 1170 (later described) may have a smaller thickness than a region of oral appliance body portion 1110 not covered with support portion 1170. In that case, the region of oral appliance body portion 1110 covered with support portion 1170 is less likely to become too thick, and the wearing feel is improved.

Oral appliance body portions 1110 according to this embodiment are each preferably mainly made of a thermoplastic resin composition. The thermoplastic resin composition is just required to contain thermoplastic resin and may further contain, for example, a filler and/or an additive in addition to the thermoplastic resin insofar as the object and the advantageous effects of the present invention are not impeded. Moreover, for example, a metal wire or plate for increasing the strength may be buried in a product of the thermoplastic resin composition.

An amount of the thermoplastic resin composition to a total mass of oral appliance body portion 1110 is preferably 50mass% to 100mass%, more preferably 60mass% to 100mass%, and particularly preferably 80mass% to 100mass%. When the amount of the thermoplastic resin composition is within the above-mentioned range, the total mass of oral appliance 1100 is reduced, and the wearing feel can be easily improved. Furthermore, it is easier to give oral appliance body portions 1110 with proper hardness and to improve the wearing feel.

A flexural modulus of the thermoplastic resin composition contained in oral appliance body portion 1110 is preferably 50 to 3000 MPa, more preferably 50 to 2000 MPa, and even more preferably 200 to 1800 MPa. The flexural modulus is measured in conformity with JIS T6501. When the flexural modulus of the thermoplastic resin composition is lower than or equal to 500 MPa, oral appliance body portion 1110 is especially easier to follow the shape of the tooth row. On the other hand, when the flexural modulus of the thermoplastic resin composition is higher than or equal to 1000 MPa, the strength of oral appliance body portion 1110 is especially increased.

Tensile strength of oral appliance body portion 1110 is preferably higher than or equal to 10 N and lower than or equal to 1000N and more preferably 100 to 800 N. When the tensile strength is within the above-mentioned range, oral appliance body portion 1110 is easier to increase followability to the shape of the tooth row. The tensile strength indicates strength at which the upper-jaw oral appliance body portion tears when a hole of φ1.5 mm is formed in a tooth No. 6 in a tooth row of the upper-jaw oral appliance body portion of the oral appliance (thickness of 3 mm) fabricated using a Nission dental model and a tensile test is performed by applying force in a direction toward the molar teeth (rearward).

Here, examples of the thermoplastic resin contained in the thermoplastic resin composition include polyester resin, polycarbonate resin, polyamide resin, (meth)acrylic resin, and so on. However, types of the thermoplastic resin are not limited to those examples. In this Description, "(meth)acrylic" indicates acrylic, methacrylic. or both of them, and "(meth)acrylate" indicates acrylatc, methacrylate. or both of them.

The polyester resin is a polycondensation product of polycarboxylic acid such as dicarboxylic acid, for example, and polyalcohol such as diol, for example. Examples of the polyester resin include polyethylene terephthalate (PET), polypropylene terephthalate, polybutylene terephthalate, and so on.

The polycarbonate resin can be given as known resin.

Examples of the polyamide resin include aliphatic polyamide resin (including alicyclic polyamide resin), semiaromatic polyamide resin, and aromatic polyamide resin. The aliphatic polyamide resin is resin including, as a main component, a structural unit containing an amide bond and not containing an aromatic ring (namely, an amide-bond-containing structural unit not containing the aromatic ring). The wording "including, as a main component, an amide-bond-containing structural unit not containing the aromatic ring" indicates that the resin includes 80mol% or more and preferably 90 to 100mol% of the amide-bond-containing structural unit not containing the aromatic ring to a total number of moles of the amide-bond containing structural unit.

Examples of the aliphatic polyamide resin (including the alicyclic polyamide resin) include resins resulting from polycondensation reaction of aliphatic dicarboxylic acids with carbon atomic numbers 2 to 14 and aliphatic diamines with carbon atomic numbers 4 to 12; resins resulting from polycondensation reaction of aliphatic amino carboxylic acids with carbon atomic numbers 6 to 12; resins resulting from ring-opening polymerization reaction of lactam with carbon atomic numbers 6 to 12; resins resulting from polycondensation reaction of aliphatic dicarboxylic acids with carbon atomic numbers 2 to 14 and diamines including one or more alicyclic structures; resins resulting from polycondensation reaction of aliphatic dicarboxylic acids including one or more alicyclic structures and aliphatic diamines with carbon atomic numbers 4 to 12; resins resulting from polycondensation reaction of dicarboxylic acids including one or more alicyclic structures and diamines including one or more alicyclic structures, and so on.

Specific examples of the above-mentioned aliphatic polyamide resins include polyamide 6, polyamide 66, polyamide 4·6, polyamide 6·10, polyamide 6·12, polyamide 6·14, polyamide 6·13, polyamide 6·15, polyamide 6·16, polyamide 9·2, polyamide 9·10, polyamide 9·12, polyamide 9·13, polyamide 9·14, polyamide 9·15, polyamide 6·16, polyamide 9·36, polyamide 10·10, polyamide 10·12, polyamide 10·13, polyamide 10·14, polyamide 11, polyamide 12, polyamide 12·10, polyamide 12·12, polyamide 12·13, polyamide 12·14, and copolymers of the above-mentioned polyamides. The aliphatic polyamide resins may be commercially available ones. Examples of the commercially available aliphatic polyamide resins include Torogamid CX7323 made by Daicel-Degussa Ltd., and so on. Among the above-mentioned examples, polyamide 11, polyamide 12, and the copolymers of aliphatic dicarboxylic acids and diamines including the alicyclic structures are especially preferable.

The semiaromatic polyamide resin is polyamide resin resulting from polycondensation of a dicarboxylic acid component and a diamine component and including the aromatic ring and the aliphatic structure.

A dicarboxylic acid structural unit constituting the semiaromatic polyamide resin preferably includes a terephthalic acid structural unit. An amount of the terephthalic acid structural unit to total 100mol% of the dicarboxylic acid structural unit constituting the semiaromatic polyamide resin is preferably 35 to 100mol% and more preferably 40 to 100mol%.

Furthermore, the dicarboxylic acid structural unit constituting the semiaromatic polyamide resin preferably includes the terephthalic acid structural unit, an isophthalic acid structural unit, and/or an aliphatic dicarboxylic acid structural unit with any of carbon atomic numbers 4 to 10.

Assuming that a total of the terephthalic acid structural unit, the isophthalic acid structural unit, and the aliphatic dicarboxylic acid structural unit with any of carbon atomic numbers 4 to 10 is 100mol%, the content of the terephthalic acid structural unit is preferably 35 to 50mol% and more preferably 40 to 50mol%. The content of the isophthalic acid structural unit is preferably 25 to 40mol% and more preferably 30 to 40mol%. The content of the aliphatic dicarboxylic acid structural unit with any of carbon atomic numbers 4 to 10 is preferably 15 to 35mol% and more preferably 20 to 30mol%.

A molar ratio of the terephthalic acid structural unit to the isophthalic acid structural unit is preferably 65/35 to 50/50. A molar ratio of the aliphatic dicarboxylic acid structural unit to the isophthalic acid structural unit is preferably 30/70 to 50/50. When the semiaromatic polyamide resin includes the aliphatic dicarboxylic acid structural unit within the above-mentioned range, the semiaromatic polyamide resin becomes amorphous, and formability is improved. On the other hand, the isophthalic acid structural unit acts to improve the formability while heat resistance of the semiaromatic polyamide resin is maintained.

The dicarboxylic acid structural unit constituting the semiaromatic polyamide resin may further include, as required, a structural unit derived from different dicarboxylic acid from the above-mentioned examples of the dicarboxylic acid. Examples of the different dicarboxylic acid include other aromatic dicarboxylic acids such as 2-methyl terephthalic acid or naphthalene dicarboxylic acid; furandicarboxylic acid such as 2,5-furandicarboxylic acid; alicyclic dicarboxylic acids such as 1,4-cyclohexane dicarboxylic acid and 1,3-cyclohexane dicarboxylic acid, and so on.

A diamine structural unit constituting the semiaromatic polyamide resin preferably includes an aliphatic diamine structural unit with any of carbon atomic numbers 4 to 12. The aliphatic diamine may be a linear aliphatic diamine or a chain aliphatic diamine with a side chain. Examples of the aliphatic diamine includes linear aliphatic diamines such as 1,4-diaminobutane, 1,6-diaminohexane, 1,7-diaminoheptane, 1,8-diaminooctane, 1,9-diaminononane, 1,10-diaminodecane, 1,11-diaminoundecane, and 1,12-diaminododecane; and chain aliphatic diamines with side chains, such as 2-methyl-1,5-diaminopetane, 2-methyl-1,6-diaminohexane, 2-methyl-1,7-diaminoheptane, 2-methyl-1,8-diaminooctane, 2-methyl-1,9-diaminononane, 2-methyl-1,10-diaminodecane, and 2-methyl-1,11-diaminundecane. The aliphatic diamine is preferably diamine with any of carbon atomic numbers 6 to 9 and more preferably hexamethylenediamine or nonanediamine.

An amount of the aliphatic diamine structural unit to total 100mol% of the diamine structural unit constituting the semiaromatic polyamide resin is preferably 60 to 100mol% and more preferably 80 to 100mol%.

The diamine structural unit constituting the semiaromatic polyamide resin may further include, as required, a structural unit derived from different diamine from the above-mentioned aliphatic diamine. Examples of the different diamine include aromatic diamines such as metaxylenediamine; and alicyclic diamines such as 1,4-cyclohexanediamine and 1,3-cyclohexanediamine.

Examples of the aromatic polyamide resin include para and meta amides, and so on.

Examples of the (meth)acrylic resin includes a (meth)acrylic thermoplastic elastomer. The (meth)acrylic thermoplastic elastomer is particularly preferably a block copolymer including a hard block and a soft block that are different in glass transition temperature (Tg).

In the block copolymer, Tg of the hard block is preferably higher than Tg of the soft block. Tg of the hard block is preferably 30°C to 200°C, more preferably 60°C to 140°C, and particularly preferably 60°C to 120°C. Tg of the soft block is preferably -100°C to 0°C, more preferably -80°C to -20°C, and particularly preferably -40°C to -50°C.

In the above-mentioned block copolymer, each of the hard block and the soft block preferably includes a unit derived from alkyl (meth)acrylate (also referred to as an "alkyl (meth)acrylate unit". In this case, the carbon number of an alkyl group contained in the alkyl (meth)acrylate unit of the hard block (also referred to as the "carbon number C1" hereinafter) is preferably smaller than the carbon number of an alkyl group contained in the alkyl (meth)acrylate unit of the soft block (also referred to as the "carbon number C2" hereinafter).

The carbon number of an alkyl portion contained in the alkyl (meth)acrylate unit of the hard block (the "carbon number C1") is preferably 1 to 6, more preferably 1 to 3, even more preferably 1 or 2, and particularly preferably 1. Furthermore, a (meth)acrylate portion is preferably methacrylate. In other words, the hard block particularly preferably includes a methyl methacrylate unit.

The carbon number of an alkyl portion contained in the alkyl (meth)acrylate unit of the soft block (the "carbon number C2") is just required to be greater than the carbon number C1. A difference between the carbon number C1 and the carbon number C2 is preferably greater than or equal to 1, more preferably 1 to 6, even more preferably 2 to 5, and particularly preferably 2 to 4.

Furthermore, a (meth)acrylate portion is preferably acrylate. The soft block particularly preferably includes a butyl acrylate unit.

In the above-mentioned block copolymer, a mass ratio of the hard block to total mass of the hard block and the soft block is selected such that the flexural modulus of the (meth)acrylic thermoplastic elastomer falls within the above-mentioned range. Thus, the above-mentioned mass ratio is preferably 5 to 95mass%, more preferably 10 to 90mass%, and particularly preferably 15 to 85mass%.

The block copolymer includes preferably 80 to 100mass%t and more preferably 90 to 100mass% of the above-mentioned hard block and soft block in total.

Mass average molecular weight (Mw) of the (meth)acrylic resin (the above-mentioned block copolymer) is selected as appropriate depending on the flexural modulus and is preferably 10000 to 1000000, more preferably 20000 to 800000, and particularly preferably 50000 to 500000. Mass average molecular weight (Mw) of the hard block is preferably 10000 to 1000000, more preferably 20000 to 800000, and particularly preferably 50000 to 500000. Mass average molecular weight (Mw) of the soft block is preferably 10000 to 1000000, more preferably 20000 to 800000, and particularly preferably 50000 to 500000. The above-mentioned mass average molecular weight (Mw) is a value measured with gel permeation chromatography (GPC) and is given as a value in terms of polymethyl methacrylate.

### (Support Portions)

Support portions 1170 of oral appliance 1100 according to this embodiment are constituted by a pair of left and right upper support portions 1171 and a pair of left and right lower support portions 1172. Upper support portions 1171 are arranged to cover upper-jaw oral appliance body portion 1111 on left and right molar tooth sides, namely to cover surfaces of upper-jaw oral appliance body portion 1111, the surfaces being opposite to other surfaces fitted to molar teeth. On the other hand, lower support portions 1172 are arranged to cover lower-jaw oral appliance body portion 1112 on left and right molar tooth sides, namely to cover surfaces of lower-jaw oral appliance body portion 1112, the surfaces being opposite to other surfaces fitted to molar teeth.

Shapes of support portions 1170 are not limited to particular ones insofar as the support portions 1170 can support positioning members 1160 described later. The shapes of the support portions may be vertically and horizontally symmetric or not symmetric. Support portions 1170 (upper support portions 1171 and lower support portions 1172) may be arranged only between oral appliance body portions 1110 and positioning members 1160, namely on surfaces of oral appliance body portions 1110 only on outer lateral sides of the molar teeth. More preferably, however, both upper support portions 1171 and lower support portions 1172 have arch-shaped structures covering inner lateral surfaces, bottom or upper surfaces, and outer lateral surfaces of the molar teeth. When support portions 1170 have arch shapes, it is easier to distribute the load and to firmly support the positioning members 1160. Widths of support portions 1170 are not limited to particular values and may be selected as appropriate in accordance with widths of upper holding portion 1130 and lower holding portion 1140 of positioning members 1160.

A thickness of each of support portions 1170 is not limited to a particular value, but it is preferably set such that, in a state of the user wearing oral appliance 1100, support portion 1170 does not excessively protrude toward a cheek side, a tongue side, and a lower or upper jaw side facing support portion 1170. As illustrated in FIGS. 1, 2A, and 2B, it is preferable that a level difference or a gap will not be generated between a region where support portion 1170 is arranged and a region where support portion 1170 is not arranged.

Support portions 1170 contain a material with a different flexural modulus from upper-jaw oral appliance body portion 1111 and lower-jaw oral appliance body portion 1112. For example, support portions 1170 may contain a material with a higher flexural modulus or a material with a lower flexural modulus than the thermoplastic resin composition contained in upper-jaw oral appliance body portion 1111 and lower-jaw oral appliance body portion 1112.

A difference between the flexural modulus of oral appliance body portions 1110 and the flexural modulus of support portions 1170 is preferably greater than or equal to 100 MPa and smaller than or equal to 1600 MPa. The difference between both the flexural moduli is more preferably smaller than or equal to 1200 MPa, even more preferably smaller than or equal to 800 MPa, and still even more preferably smaller than or equal to 600 MPa. On the other hand, the difference between both the flexural moduli is more preferably greater than or equal to 50 MPa.

The flexural moduli of oral appliance body portions 1110 and support portions 1170 are measured in conformity with JIS T6501. With the flexural modulus of oral appliance body portions 1110 and the flexural modulus of support portions 1170 being different from each other, even if load is applied to oral appliance body portions 1110 from positioning members 1160 when oral appliance 1100 is put on, force exerted on oral appliance 1100 is relieved by support portions 1170 or oral appliance body portions 1110, and the wearing feel can be improved while damage and bending are suppressed. In addition, the rearward displacement of lower-jaw oral appliance body portion 1112 can also be suppressed.

Each of support portions 1170 may contain, for example, various types of fillers and/or additives together with a high elastic material and a low elastic material insofar as the object and the advantageous effects of the present invention are not impeded. However, an amount of the high elastic material and the low elastic material to total mass of support portion 1170 is preferably 50mass% to 100mass%, more preferably 60mass% to 100mass%, and particularly preferably 80mass% to 100mass%. When the amount of the high elastic material and the low elastic material is within the above-mentioned range, support portion 1170 is sufficiently hardened and can more easily support positioning members 1160.

The high elastic material and the low elastic material are not limited to particular ones insofar as those materials can realize the above-mentioned difference in the flexural modulus between oral appliance body portions 1110 and support portions 1170. Examples of the high elastic material include metal and (meth)acrylic resin. Materials of support portions 1170 are not limited to the above-mentioned examples.

The metal contained in support portions 1170 is just required not to substantially affect the human body. Examples of the metal include titanium, iron (such as steel containing stainless), gold, silver, platinum, cobalt, chromium, etc. and alloys of those metals. Corrosion resistance of the metal may be increased with a chemically formed coating, for example.

The (meth)acrylic resin is just required to be resin that mainly includes a unit derived from (meth)acrylate (also referred to as a "(meth)acrylatc unit" hereinafter). The (meth)acrylic resin contains preferably 50mass% to 100mass%, more preferably 60mass% to 100mass%, and particularly preferably 80mass% to 100mass% of the (meth)acrylate unit to total mass of structural units of the (meth)acrylic resin. The (meth)acrylic resin may be, for example, a homopolymer of acrylate, a homopolymer of methacrylate, or a copolymer of acrylate and methacrylate.

The (meth)acrylate unit is preferably an alkyl (meth)acrylate unit. The carbon number of an alkyl portion of the alkyl (meth)acrylate unit is preferably 1 to 6, more preferably 1 to 3, even more preferably 1 or 2, and particularly preferably 1.

A (meth)acrylate portion of the alkyl (meth)acrylate unit is preferably methacrylate and includes preferably 50mass% to 100mass%, more preferably 60mass% to 100mass%, and particularly preferably 80mass% to 100mass% to total mass of the structural units of the (meth)acrylic resin.

The (meth)acrylic resin may contain a structural unit other than the (meth)acrylate unit insofar as the object and the advantageous effects of the present invention are not impeded. Examples of the structural unit other than the (meth)acrylate unit includes a structural unit derived from (meth)acrylic acid (preferably, methacrylic acid) and a structural unit derived from α- olefin (preferably, ethylene, propylene, or butylene).

The (meth)acrylic resin is preferably polymethyl methacrylate (PMMA) or a methyl methacrylate - methacrylic acid copolymer (MMA-MAA copolymer) and is particularly preferably PMMA.

Mass average molecular weight (Mw) of the (meth)acrylic resin is preferably 10000 to 1000000, more preferably 20000 to 800000, and particularly preferably 50000 to 500000. The mass average molecular weight (Mw) is a value measured with gel permeation chromatography (GPC) and is given as a value in terms of polymethyl methacrylate.

A density of the material of support portions 1170 is preferably smaller than 1.5 kg/m³, more preferably 0.8 to 1.4 kg/m³, and even more preferably smaller than 0.8 to 1.3 kg/m³. When the density of the material of support portions 1170 is smaller than 1.5 kg/m³, the weight of oral appliance 1100 can be reduced, and feel in use can be easily improved.

### (Positioning Members)

Positioning members 1160 are members for adjusting the relative positions of upper-jaw oral appliance body portion 1111 and lower-jaw oral appliance body portion 1112, and for suppressing the rearward displacement of lower-jaw oral appliance body portion 1112. In this embodiment, each of positioning members 1160 includes upper holding portion 1130 attached to an outer lateral surface of upper-jaw oral appliance body portion 1111 with upper support portion 1171 interposed therebetween, lower holding portion 1140 attached to an outer lateral surface of lower-jaw oral appliance body portion 1112 with lower support portion 1172 interposed therebetween, and coupling portion 1150 coupling upper holding portion 1130 and lower holding portion 1140.

A structure of upper holding portion 1130 is not limited to a particular one insofar as upper holding portion 1130 can be joined to upper support portion 1171 and can hold coupling portion 1150 described later. As illustrated in FIG. 1, for example, upper holding portion 1130 may have a structure including upper shaft 1132 extending outward from upper support portion 1171 and upper flange portion 1134 positioned further outward of upper shaft 1132. One end of upper shaft 1132 in the form of a column is supported by upper support portion 1171. A method of joining upper support portion 1171 and upper shaft 1132 is not limited to a particular one. For example, upper shaft 1132 may be supported by being inserted into a through-hole that is formed in upper support portion 1171, or may be buried in upper support portion 1171.

A structure of lower holding portion 1140 is also not limited to a particular one insofar as lower holding portion 1140 can be joined to lower support portion 1172 and can hold coupling portion 1150 described later. As illustrated in FIG. 1, for example, lower holding portion 1140 may have a structure including lower shaft 1142 extending outward from lower support portion 1172 and lower flange portion 1144 positioned further outward of lower shaft 1142. One end of lower shaft 1142 in the form of a column is supported by lower support portion 1172. A method of joining lower support portion 1172 and lower shaft 1142 is similar to the method of joining upper support portion 1171 and upper shaft 1132.

A structure of coupling portion 1150 is not limited to a particular one insofar as coupling portion 1150 can couple upper holding portion 1130 and lower holding portion 1140 and can hold upper holding portion 1130 and lower holding portion 1140 while keeping a certain distance therebetween. In positioning member 1160 illustrated in FIG. 1, coupling portion 1150 includes cylindrical outer 1152 with one end supported by upper shaft 1132 of upper holding portion 1130, inner 1154 with one end supported by lower shaft 1142 of lower holding portion 1140 and the other end slidably received in outer 1152, and a stopper (not illustrated) that is arranged inside outer 1152 and determines an extension/contraction stroke of inner 1154. Coupling portion 1150 has such a structure that the inner 1154 is slidably received in the outer 1152 to be capable of extending and contracting. Furthermore, the distance between upper holding portion 1130 and lower holding portion 1140 and hence a positional relation between upper-jaw oral appliance body portion 1111 and lower-jaw oral appliance body portion 1112 can be adjusted in accordance with the position of the stopper.

In positioning member 1160 according to this embodiment, lower holding portion 1140 is arranged forward of upper holding portion 1130. Stated in another way, positioning member 1160 has a structure of pushing lower-jaw oral appliance body portion 1112 forward from a rear side and restricting the rearward displacement of lower-jaw oral appliance body portion 1112. On the other hand, because inner 1154 can be withdrawn from outer 1152 over a certain range, movement of the lower jaw is hardly impeded, and the wearing feel of the user can be easily made less uncomfortable. A restriction position and a sliding stroke of the lower jaw are adjusted in accordance with the position of the stopper (not illustrated) of coupling portion 1150. Moreover, the structure of coupling portion 1150 is not limited to the above-described one and may be given as a structure capable of extending and contracting in multiple stages.

Positioning member 1160 may be made of the same material in the entirety or made of different materials. Examples of the material(s) of positioning member 1160 include metal materials such as titanium, iron (such as steel containing stainless), gold, silver, platinum, cobalt, chromium, and alloys of those metals, resin materials (such as polycarbonate resin) with the flexural moduli of higher than or equal to 1000 MPa and lower than or equal to 3000 MPa, those flexural moduli being measured in conformity with JIS T6501, and so on. Corrosion resistance of the metal materials may be increased with a chemically formed coating, for example.

### (Method of Manufacturing Oral Appliance According to Embodiment 1)

A method of manufacturing oral appliance 1100 according to this embodiment is not limited to a particular one. For example, oral appliance body portions 1110 and support portions 1170 may be fabricated by preparing sheets containing the thermoplastic resin composition and sheets containing the above-described high elastic material and low elastic material, and by molding the sheets with heat molding (for example, suction molding, pressure molding, or suction and heat molding). Alternatively, the above-mentioned components may be formed by pouring the thermoplastic resin composition, the high elastic material, etc. into molds, or may be fabricated by laser processing, for example. In this embodiment, the above-mentioned components are particularly preferably fabricated by milling or 3D printing. Those methods enable oral appliance body portions 1110 and support portions 1170 to be fabricated with high accuracy. Furthermore, with the milling or the 3D printing, the machining or the processing can be performed even when a material with high transparency to visible light is used for oral appliance body portions 1110 and so on. Oral appliance body portions 1110 and support portions 1170 may be fabricated by the same method or different methods.

For example, when oral appliance body portions 1110 and support portions 1170 are fabricated by the milling, each oral appliance body portion 1110 and each support portions 1170 may be fabricated separately, and individual parts may be joined by fusion bonding or with an adhesive, for example. Alternatively, it is also possible to previously prepare a stacked body in which the thermoplastic resin composition forming oral appliance body portion 1110 and the high elastic material and the low elastic material forming support portion 1170 are stacked one above the other, and to perform the milling on the stacked body.

When oral appliance body portions 1110 and support portions 1170 are fabricated by the 3D printing, they may be produced from the thermoplastic resin composition, the high elastic material, etc. by using a known 3D printer. In this case as well, each oral appliance body portions 1110 and each support portion 1170 may be fabricated separately, and individual parts may be joined by fusion bonding or with an adhesive, for example, or may be formed integrally.

A method of fabricating each of positioning members 1160 is selected as appropriate depending on the material, and positioning member 1160 can be fabricated by a known metal machining method or a known resin processing method. A method of joining support portion 1170 and positioning member 1160 is not limited to a particular one and is selected as appropriate in conformity with the method of fabricating support portion 1170 and so on. For example, a through-hole may be formed in support portion 1170 that has been fabricated in advance, and upper shaft 1132 or lower shaft 1142 may be inserted into the through-hole. Alternatively, upper shaft 1132 or lower shaft 1142 may be arranged in place and, in such a state, upper shaft 1132 or lower shaft 1142 may be buried in support portion 1170 by 3D printing or heat molding, for example.

### 2. Embodiment 2

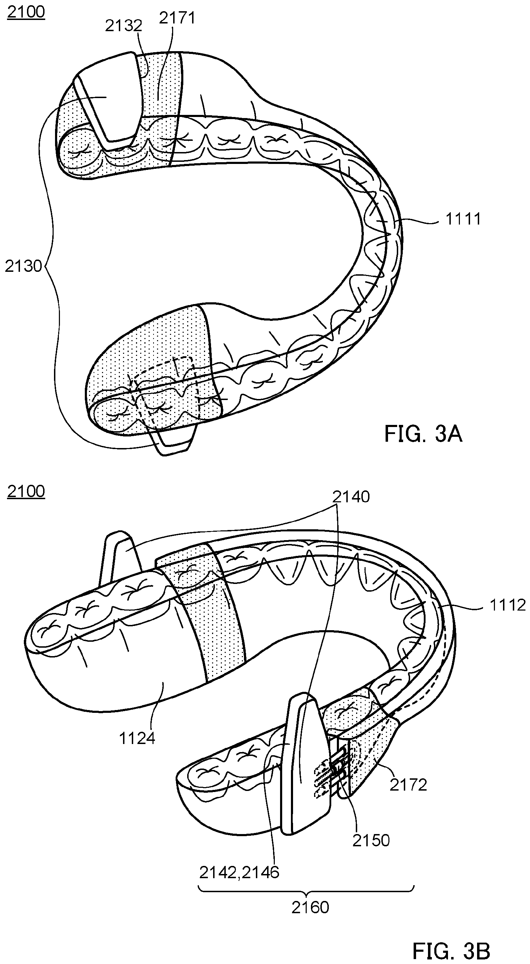

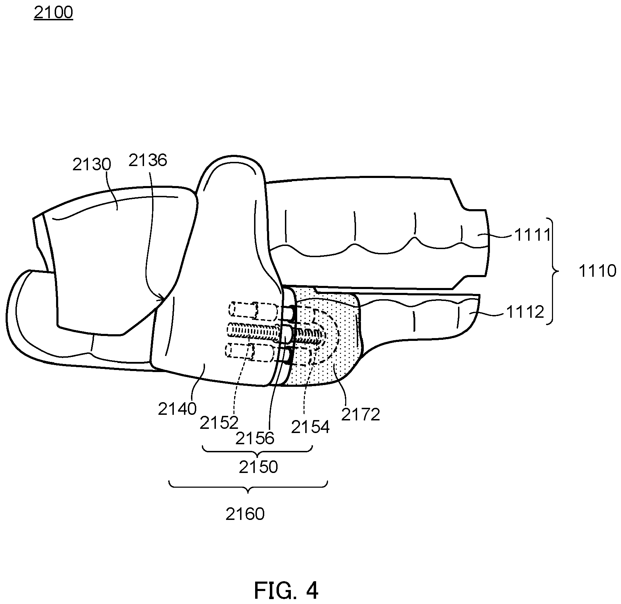

FIG. 3A is a schematic perspective view illustrating an upper-jaw side structure of oral appliance 2100 according to Embodiment 2 of the present invention, and FIG. 3B is a schematic perspective view illustrating a lower-jaw side structure of the oral appliance 2100. FIG. 4 is a side view of the oral appliance 2100 in the mouth closed state. As illustrated in FIGS. 3A, 3B, and 4, oral appliance 2100 according to this embodiment includes a pair of oral appliance body portions 1110 constituted by upper-jaw oral appliance body portion 1111 and lower-jaw oral appliance body portion 1112, positioning members constituted by a pair of left and right stoppers 2130 arranged on upper-jaw oral appliance body portion 1111 and by a pair of left and right movable wings 2160 arranged on lower-jaw oral appliance body portion 1112, and support portions constituted by a pair of left and right upper support portions 2171 that cover parts of upper-j aw oral appliance body portion 1111 and that support stoppers 2130, and by a pair of left and right lower support portions 2172 that cover parts of lower-jaw oral appliance body portion 1112 and that support movable wings 2160.

In oral appliance 2100 according to this embodiment, the rearward displacement of lower-jaw oral appliance body portion 1112 is suppressed by movable wings 2160 (specifically, the wings 2140 described later) and stoppers 2130 of the positioning members abutting on each other. As a result, an airway of the user is kept wide, and apnea or snoring is cured during sleep.

In oral appliance 2100 according to this embodiment as well, the support portions (upper support portions 2171 and lower support portions 2172) contain a material with a different flexural modulus from the pair of oral appliance body portions 1110. Furthermore, the support portions (upper support portions 2171 and lower support portions 2172) is arranged to cover only parts of oral appliance body portions 1110. For example, when the flexural modulus of the material of the support portions (upper support portions 2171 and lower support portions 2172) is higher than that of oral appliance body portions 1110, oral appliance body portions 1110 are easier to come into close contact with the tooth rows of the user, and the wearing feel becomes more comfortable. The support portions (upper support portions 2171 and lower support portions 2172) are less susceptible to deformation and can support the positioning members (stoppers 2130 and movable wings 2160) at the desired positions. In addition, even when load is applied, the support portions are less susceptible to deformation and damage. As a result, oral appliance 2100 according to this embodiment can be stably used for a long period. On the other hand, when the flexural modulus of the material of the support portions (upper support portions 2171 and lower support portions 2172) is lower than that of oral appliance body portions 1110, the support portions relieve force applied to oral appliance 2100, whereby the wearing feel is improved while damage and bending are suppressed.

The pair of oral appliance body portions 1110 (upper-jaw oral appliance body portion 1111 and lower-jaw oral appliance body portion 1112) in this embodiment are similar to the pair of oral appliance body portions 1110 in Embodiment 1. Hence those body portions are denoted by the same reference signs as in FIG. 1 and so on, and description of those body portions is omitted. The support portions and the positioning members of oral appliance 2100 according to this embodiment will be described below.

### (Support Portions)

The support portions of oral appliance 2100 according to this embodiment are constituted by a pair of left and right upper support portions 2171 and a pair of left and right lower support portions 2172. Upper support portions 2171 are arranged to cover upper-jaw oral appliance body portion 1111 on left and right molar tooth sides, namely to cover surfaces of upper-jaw oral appliance body portion 1111, the surfaces being opposite to other surfaces fitted to molar teeth. On the other hand, lower support portions 2172 are arranged to cover lower-jaw oral appliance body portion 1112 on left and right molar tooth sides, namely to cover surfaces of lower-jaw oral appliance body portion 1112, the surfaces being opposite to other surfaces fitted to molar teeth.

Shapes of upper support portions 2171 are not limited to particular ones insofar as upper support portions 2171 can support stoppers 2130 of the positioning members described later. The shapes of upper support portions 2171 may be horizontally symmetric or not symmetric. Upper support portions 2171 may be arranged, for example, only between upper-jaw oral appliance body portion 1111 and support stoppers 2130 of the positioning members, namely on surfaces of upper-jaw oral appliance body portion 1111 only on outer lateral sides of the molar teeth. More preferably, upper support portions 2171 have arch-shaped structures covering inner lateral surfaces, bottom surfaces, and outer lateral surfaces of the molar teeth. When upper support portions 2171 have arch shapes, load can be more easily distributed, and upper support portions 2171 are less susceptible to damage even when the load is applied from stoppers 2130. Widths of upper support portions 2171 are not limited to particular values and may be selected as appropriate in accordance with widths of stoppers 2130 of the positioning members.

A thickness of each of upper support portions 2171 is preferably set such that, in a state of the user wearing oral appliance 2100, upper support portion 2171 does not excessively protrude toward a cheek side, a tongue side, and a lower jaw side facing upper support portions 2171. As illustrated in FIG. 3A, upper support portion 2171 is preferably formed not to generate a level difference or a gap between upper support portion 2171 and upper-jaw oral appliance body portion 1111.

Shapes of lower support portions 2172 are not limited to particular ones insofar as lower support portions 2172 can support the positioning members (adjustment portions 2150 of movable wings 2160) described later . The shapes of lower support portions 2172 may be horizontally symmetric or not symmetric. Lower support portions 2172 may be arranged on lower-jaw oral appliance body portion 1112 only on outer lateral sides of the molar teeth. However, lower support portions 2172 preferably have arch-shaped structures covering inner lateral surfaces, upper surfaces, and outer lateral surfaces of the molar teeth. When lower support portions 2172 have arch shapes, adjustment portions 2150 of movable wings 2160 can be firmly supported by lower support portions 2172. Widths of lower support portions 2172 are not limited to particular values and may be selected as appropriate in accordance with widths of adjustment portions 2150 of movable wings 2160 of the positioning members.

Each of lower support portions 2172 are preferably formed such that, in the state of the user wearing oral appliance 2100, lower support portion 2172 does not excessively protrude toward a tongue side and an upper jaw side facing lower support portions 2172. On the other hand, lower support portion 2172 preferably has, on the outer lateral surface side of the molar teeth (namely, on the cheek side), a thickness allowing part of adjustment portion 2150 of movable wing 2160 of the positioning member to be sufficiently buried in lower support portion 2172. Furthermore, as illustrated in FIG. 3B, lower support portion 2172 is formed such that part of adjustment portion 2150 of movable wing 2160 of the positioning member protrudes rearward of lower support portion 2172.

A flexural modulus of the support portions (upper support portions 2171 and lower support portions 2172) in this embodiment is just required to be different from that of upper-jaw oral appliance body portion 1111 and lower-jaw oral appliance body portion 1112. For example, the support portions (upper support portions 2171 and lower support portions 2172) may contain a high elastic material with a higher flexural modulus or a low elastic material with a lower flexural modulus than the thermoplastic resin composition forming upper-jaw oral appliance body portion 1111 and lower-jaw oral appliance body portion 1112. When the support portions contain the high elastic material, the support portions are sufficiently hardened, and the positioning members (stoppers 2130 and movable wings 2160) can be more easily supported at the desired positions. Materials contained in the support portions are similar to those contained in the above-described support portions in Embodiment 1, and hence detailed description of those materials is omitted here.

### (Positioning Members)

The positioning members are members for adjusting the relative positions of upper-jaw oral appliance body portion 1111 and lower-jaw oral appliance body portion 1112. The positioning members in this embodiment include a pair of left and right stoppers 2130 attached to the outer lateral surface of upper-jaw oral appliance body portion 1111 with upper support portions 2171 interposed therebetween, and movable wings 2160 attached to the outer lateral surface of lower-jaw oral appliance body portion 1112 with lower support portions 2172 interposed therebetween, each of movable wings 2160 including wing 2140 and adjustment portion 2150.

In the positioning members, rear surfaces 2142 of wings 2140 of movable wings 2160 and front surfaces 2132 of stoppers 2130 abut on each other, whereby the position of the lower jaw of the user is adjusted. More specifically, the rearward displacement of lower-jaw oral appliance body portion 1112 is limited in an occlusal state with the wings 2140 and the stoppers 2130 abutting on each other, and the rearward displacement of the lower jaw of the user wearing oral appliance 2100 is limited.

A structure of each of stoppers 2130 is not limited to a particular one insofar as the structure enables the stopper to abut on wing 2140 of movable wing 2160 described later. As illustrated in FIG. 3A, for example, stoppers 2130 can be formed of a pair of left and right plate-shaped members supported by upper support portions 2171 and protruding toward the low jaw from the upper jaw of the user. However, stoppers 2130 are not always required to protrude downward beyond a bottom surface of upper-jaw oral appliance body portion 1111 in a wearing state of oral appliance 2100.

As illustrated in FIG. 4, each of movable wings 2160 includes wing 2140 abutting on stopper 2130 when the user occludes the teeth while wearing upper-jaw oral appliance body portion 1111 and lower-jaw oral appliance body portion 1112, and adjustment portion 2150 adjusting the position of wing 2140. As described above, part of adjustment portion 2150 is incorporated in lower support portion 2172.

A structure of wing 2140 is not limited to a particular one insofar as the structure enables a rear surface of the wing 2140 (rear surface 2142 to be abutted on stopper 2130) to abut on front surface 2132 of stopper 2130. Wing 2140 of oral appliance 2100 illustrated in FIGS. 3A, 3B, and 4 is a plate-shaped member protruding above an upper surface of lower-jaw oral appliance body portion 1112 in the occlusal state.

Furthermore, the position of wing 2140 is adjusted in a front-rear direction by adjustment portion 2150 that is supported by lower support portion 2172. When wing 2140 is arranged at a more rear position by adjustment portion 2150, lower-jaw oral appliance body portion 1112 is moved further forward in the occlusal state.

Adjustment portion 2150 includes first screw rod 2154 having one end supported in lower support portion 2172 and including a male screw formed thereon, second screw rod 2152 having one end supported in wing 2140 and including a male screw formed thereon, and turnbuckle 2156 inserted into screw holes with which the other ends of first screw rod 2154 and second screw rod 2152 mesh. With adjustment portion 2150, the position of wing 2140 fixed to second screw rod 2152 is adjusted by inserting a rotation pin into a pin hole formed in turnbuckle 2156, rotating turnbuckle 2156, and by adjusting an amount of meshing between turnbuckle 2156 and each of first screw rod 2154 and second screw rod 2152.

Stopper 2130 and wing 2140 preferably have such shapes that stopper 2130 does not restrict movement of wing 2140 when lower-jaw oral appliance body portion 1112 is moved in a mouth opening direction during the wearing of the oral appliance. More specifically, as illustrated in FIG. 4, stopper 2130 may include inclined portion 2136 that enables lower-jaw oral appliance body portion 1112 to be easily moved in the mouth opening direction. Furthermore, as illustrated in FIG. 3B, wing 2140 may include inclined portion 2146 that enables lower-jaw oral appliance body portion 1112 to be easily moved in the mouth opening direction. In other words, stopper 2130 may include, in its front end portion, a rearward-extending inclined surface having an upper end positioned on a relatively forward side and a lower end positioned on a relatively rearward side, and wing 2140 may include, in its rear end portion, a rearward-extending inclined surface having an upper end positioned on a relatively forward side and a lower end positioned on a relatively rearward side. Inclined portion 2146 of wing 2140 abuts on stopper 2130 in the occlusal state.

Each of stopper 2130 and wing 2140 preferably contains a material with a high flexural modulus, for example, a material similar to the high elastic material forming the support portions. With stopper 2130 and wing 2140 containing the high elastic materials, strength of stopper 2130 and wing 2140 is sufficiently increased. As a result, the positions of the upper jaw and the lower jaw are less likely to shift during use of oral appliance 2100. On the other hand, adjustment portion 2150 can be made of a material similar to that of the positioning members in Embodiment 1. Thus, adjustment portion 2150 is preferably made of a metal material or a resin material (for example, polycarbonate resin) with the flexural modulus of higher than or equal to 1000 MPa and lower than or equal to 3000 MPa when measured in conformity with the JIS T6501.

Although, in the above-described description, stoppers 2130 are arranged on the upper jaw side and movable wings 2160 are arranged on the lower jaw side, movable wings 2160 may be arranged on the upper jaw side and stoppers 2130 may be arranged on the lower jaw side.

### (Method of Manufacturing Oral Appliance According to Embodiment 2)

A method of manufacturing oral appliance 2100 according to this embodiment is not limited to a particular one. For example, oral appliance body portions 1110, the support portions, stoppers 2130 and wings 2140 of the positioning members, and so on may be fabricated by preparing sheets containing the thermoplastic resin composition and sheets containing the high elastic material and the low elastic material, and by molding the sheets with heat molding (for example, suction molding, pressure molding, or suction and heat molding). Alternatively, the above-mentioned components may be formed by pouring the thermoplastic resin composition, the high elastic material, etc. into molds, or may be fabricated by laser processing, for example. In this embodiment, the above-mentioned components are particularly preferably fabricated by milling or 3D printing. Those methods enable oral appliance body portions 1110, the support portions, stoppers 2130 and wings 2140 of the positioning members, and so on to be fabricated with high accuracy. Furthermore, with the milling or the 3D printing, the machining or the processing can be performed even when a material with high transparency to visible light is used for oral appliance body portions 1110 and so on.

Oral appliance body portions 1110, the support portions, and stoppers 2130 and wings 2140 of the positioning members may be fabricated by the same method or different methods. Moreover, oral appliance body portions 1110, the support portions, stoppers 2130 of the positioning members, and so on may be fabricated separately, and individual parts may be joined by fusion bonding or with an adhesive, for example, or may be molded integrally.

A method of fabricating each of adjustment portions 2150 of movable wings 2160 is selected as appropriate depending on the material, and adjustment portion 2150 can be fabricated by a known metal machining method or a known resin processing method. A method of joining lower support portion 2172 and wing 2140 to adjustment portion 2150 is selected as appropriate in conformity with the method of forming the support portions. For example, adjustment portion 2150 may be buried in wing 2140 or lower support portion 2172 by performing the 3D printing or the heat molding, for example, in a state in which adjustment portion 2150 is arranged in place.

### 3. Embodiment 3

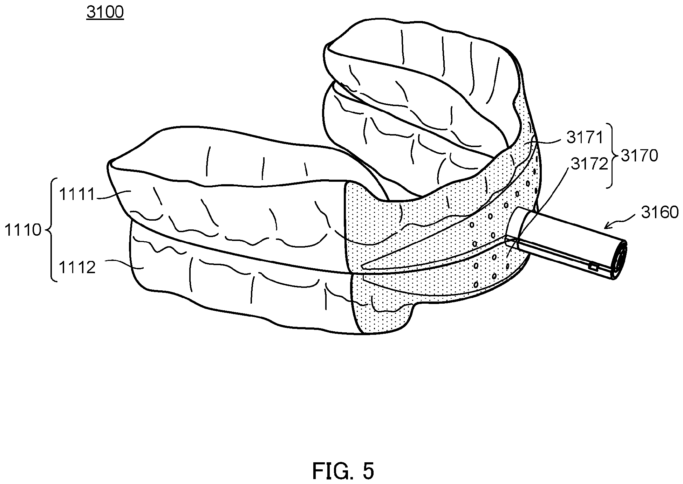

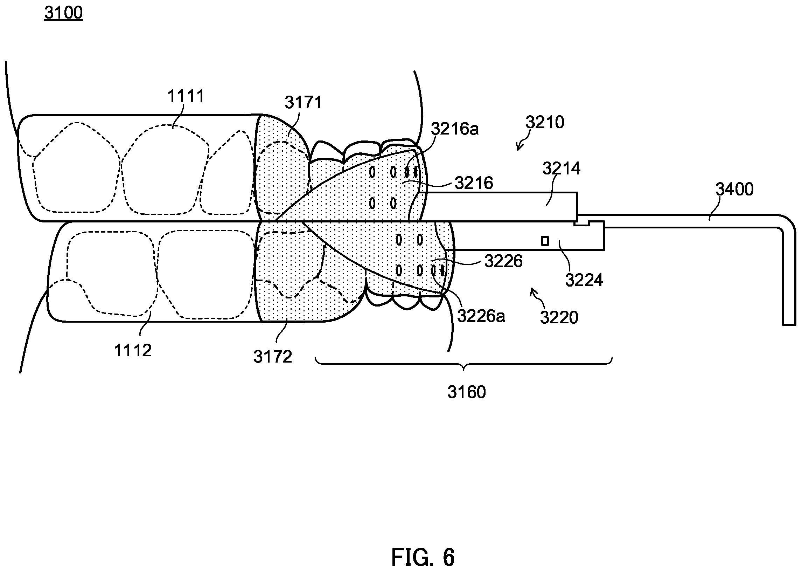

FIG. 5 is a schematic perspective view of oral appliance 3100 according to Embodiment 3 of the present invention, and FIG. 6 is a schematic side view of the oral appliance 3100 in the mouth closed state. As illustrated in FIGS. 5 and 6, oral appliance 3100 includes a pair of oral appliance body portions 1110 constituted by upper-jaw oral appliance body portion 1111 and lower-jaw oral appliance body portion 1112, a positioning member 3160 that restricts the rearward displacement of lower-jaw oral appliance body portion 1112, and support portions 3170 constituted by upper support portion 3171 that covers part of upper-jaw oral appliance body portion 1111 and that supports positioning member 3160, and a lower support portion 3172 that covers part of lower-jaw oral appliance body portion 1112 and that supports positioning member 3160.

In oral appliance 3100 according to this embodiment, the rearward displacement of lower-jaw oral appliance body portion 1112 is restricted by positioning member 3160, whereby an airway of the user is kept wide and apnea or snoring is cured during sleep.

In oral appliance 3100 according to this embodiment as well, support portions 3170 (upper support portion 3171 and lower support portion 3172) contain a material with a different flexural modulus from the pair of oral appliance body portions 1110. Furthermore, the support portions 3170 are arranged to cover only parts of oral appliance body portions 1110. For example, when support portions 3170 include high elastic material with a higher flexural modulus than oral appliance body portions 1110, oral appliance body portions 1110 are easier to come into close contact with the tooth rows of the user, and the wearing feel becomes more comfortable. Furthermore, in that case, the support portions 3170 are less susceptible to deformation and can support the positioning member 3160 at the desired position. In addition, even when load is applied to oral appliance 3100, the support portions 3170 are less susceptible to deformation and damage. As a result, oral appliance 3100 according to this embodiment can be stably used for a long period. On the other hand, when the flexural modulus of the material contained in the support portions 3170 is lower than that of oral appliance body portions 1110, the support portions 3170 relieve force applied to oral appliance 3100, whereby the wearing feel is improved while damage and bending are suppressed.

The pair of oral appliance body portions (upper-jaw oral appliance body portion 1111 and lower-jaw oral appliance body portion 1112) in this embodiment are similar to the pair of oral appliance body portions 1110 in Embodiment 1. Hence those body portions are denoted by the same reference signs as in FIG. 1 and so on, and description of those body portions is omitted. The support portions 3170 and the positioning member 3160 of oral appliance 3100 according to this embodiment will be described below.

### (Support Portions)

The support portions of oral appliance 3100 according to this embodiment are constituted by upper support portion 3171 and lower support portion 3172. Upper support portion 3171 is arranged to cover upper-jaw oral appliance body portion 1111 at least on an incisor tooth side, namely to cover a surface of upper-jaw oral appliance body portion 1111, the surface being opposite to another surface fitted to incisor teeth. On the other hand, lower support portion 3172 is arranged to cover lower-jaw oral appliance body portion 1112 at least on an incisor tooth side, namely to cover a surface of lower-jaw oral appliance body portion 1112, the surface being opposite to another surface fitted to incisor teeth.

Shapes of support portions 3170 (upper support portion 3171 and lower support portion 3172) are not limited to particular ones insofar as support portions 3170 can support positioning member 3160 (described later) on a front side of the incisor teeth. Support portions 3170 may be arranged, for example, only between oral appliance body portions 1110 and mounting portions 3216 and 3226 of positioning member 3160 described later. Alternatively, or mounting portions 3216 and 3226 of positioning member 3160 may be buried in support portions 3170.

Support portions 3170 (upper support portion 3171 and lower support portion 3172) may be arranged only on the outer lateral surfaces of upper-jaw oral appliance body portion 1111 and lower-jaw oral appliance body portion 1112. However, support portions 3170 preferably have arch-shaped structures covering not only the outer lateral surfaces, but also bottom surfaces, upper surfaces, and inner lateral surfaces of upper-jaw oral appliance body portion 1111 and lower-jaw oral appliance body portion 1112. When upper support portion 3171 and lower support portion 3172 have arch shapes, positioning member 3160 can be firmly supported by upper support portion 3171 and lower support portion 3172. Widths of the support portions (upper support portion 3171 and lower support portion 3172) are selected as appropriate in accordance with widths of mounting portions 3216 and 3226 of positioning member 3160.

Support portions 3170 in this embodiment are also just required to have a different flexural modulus from upper-jaw oral appliance body portion 1111 and lower-jaw oral appliance body portion 1112. Support portions 3170 may contain a high elastic material with a higher flexural modulus or a low elastic material with a lower flexural modulus than upper-jaw oral appliance body portion 1111 and lower-jaw oral appliance body portion 1112. For example, when support portions 3170 contain the high elastic material, support portions 3170 are sufficiently hardened, and the positioning member 3160 can be more easily supported at the desired position. Materials contained in support portions 3170 are similar to those contained in the above-described support portions in Embodiment 1, and hence detailed description of those materials is omitted here.

### (Positioning Member)

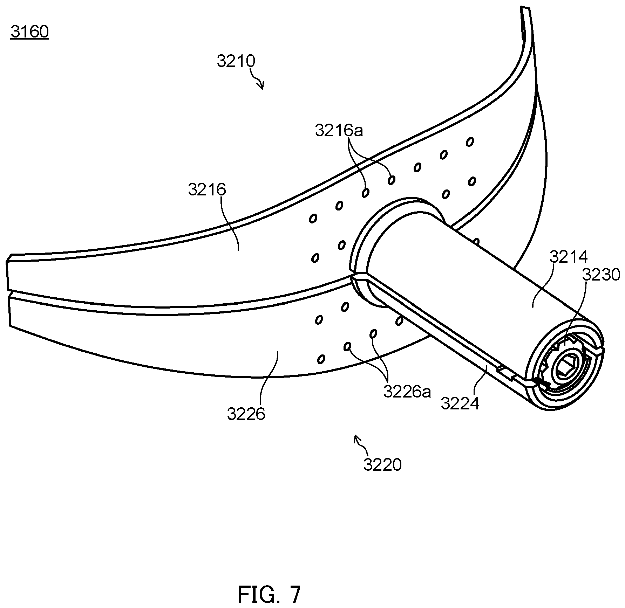

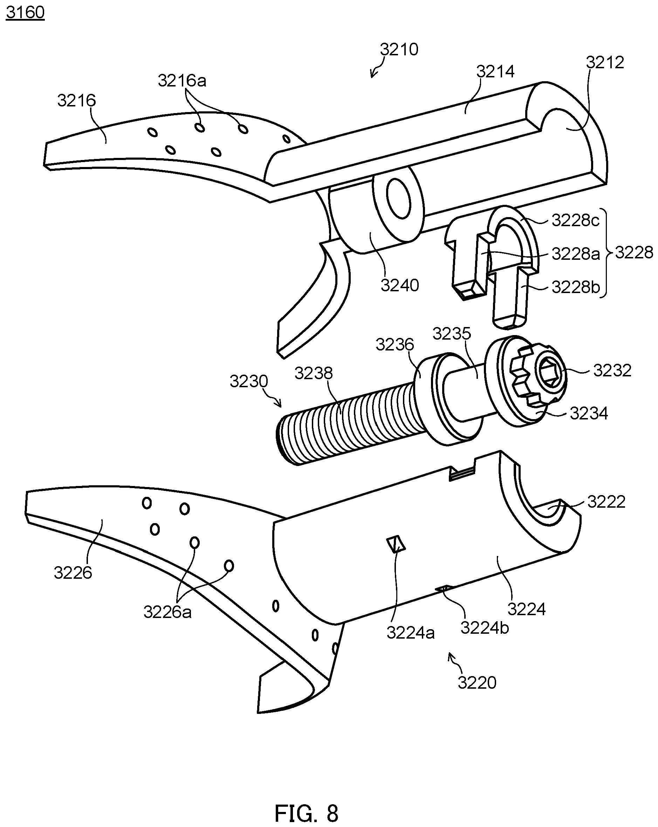

Positioning member 3160 is a member for adjusting the relative positions of upper-jaw oral appliance body portion 1111 and lower-j aw oral appliance body portion 1112. FIG. 7 is a schematic perspective view of positioning member 3160, and FIG. 8 is a schematic exploded perspective view of positioning member 3160.

As illustrated in FIGS. 6, 7, and 8, positioning member 3160 in this embodiment includes at least fixed member 3210 supported by upper support portion 3171, sliding member 3220 supported by lower support portion 3172, and male screw 3230 coupling fixed member 3210 and sliding member 3220 such that fixed member 3210 and sliding member 3220 are arranged with a predetermined gap kept therebetween.

Fixed member 3210 includes substantially semicylindrical cover portion 3214 of which surface facing sliding member 3220 defines substantially semicylindrical groove portion 3212, and sliding member 3220 includes substantially semicylindrical cover portion 3224 of which surface facing fixed member 3210 defines substantially semicylindrical groove portion 3222. When fixed member 3210 and sliding member 3220 are arranged with groove portions 3212 and 3222 facing each other, a substantially cylindrical insertion hole is formed through which male screw 3230 can be inserted.

Shapes of cover portion 3214 and cover portion 3224 are not limited to the above-described ones, and a substantially truncated conical cylindrical shape, a substantially elliptic cylindrical shape, or a substantially truncated elliptic conical cylindrical shape, for example, may be formed when both the cover portions are arranged to face each other. Furthermore, an outer shape of cover portion 3214 and an outer shape of cover portion 3224 are not always required to be substantially the same and may be different from each other insofar as the above-mentioned insertion hole can be formed.

Fixed member 3210 further includes, in addition to cover portion 3214, mounting portion 3216 for fixing fixed member 3210 to upper support portion 3171, and female screw 3240.

Cover portion 3214 may be, for example, a thin plate member formed into a semicylindrical shape. Cover portion 3214 may be formed in substantially the same thickness from a front end to a rear end that is in continuity with mounting portion 3216, or may be formed to have a greater thickness at the rear end to which stress in the left-right direction tends to be applied during the wearing of oral appliance 3100.

Mounting portion 3216 is a thin plate member extending in the left-right direction continuously from the rear end of cover portion 3214 and is formed in a curved shape following the shape of a front surface of upper-jaw oral appliance body portion 1111. Mounting portion 3216 is fixed to a front side of upper-jaw oral appliance body portion 1111 with upper support portion 3171 interposed therebetween. Mounting portion 3216 may include multiple through-holes 3216a that allow mounting portion 3216 to be deformed for easier mounting to upper support portion 3171.

Female screw 3240 is arranged at a position on an inner circumference of groove portion 3212 rearward of the above-mentioned insertion hole. In this embodiment, female screw 3240 is made of the same material as cover portion 3214 and mounting portion 3216 and is formed integrally with cover portion 3214 and so on.

Female screw 3240 may be arranged at a rear end of groove portion 3212. However, female screw 3240 is preferably arranged with a distance kept from the rear end of groove portion 3212 from the viewpoint of suppressing reduction in the wearing feel of the user, the reduction being attributable to a situation that the male screw 3230 deeply meshed with the female screw 3240 and protruding from a rear end of the above-mentioned insertion hole abuts on the tongue of the user.

Sliding member 3220 may include, in addition to cover portion 3224, mounting portion 3226 for fixing sliding member 3220 to lower support portion 3172, and U-shaped fastener 3228 that holds male screw 3230.

Cover portion 3224 may be, for example, a thin plate member formed into a semicylindrical shape. Cover portion 3224 may be formed in substantially the same thickness from a front end to a rear end that is in continuity with mounting portion 3226, or may be formed to have a greater thickness at the rear end to which stress in the left-right direction tends to be applied during the wearing of oral appliance 3100. Cover portion 3224 further includes through-holes 3224a and 3224b into which end portions of fastener 3228 can be fitted.

Mounting portion 3226 is a thin plate member extending in the left-right direction continuously from the rear end of cover portion 3224 and is formed in a curved shape following the shape of a front surface of lower-jaw oral appliance body portion 1112. Mounting portion 3226 is supported to the front surface of lower-jaw oral appliance body portion 1112 with lower support portion 3172 interposed therebetween. Mounting portion 3226 may include multiple through-holes 3226a that allow mounting portion 3226 to be deformed for easier mounting to lower support portion 3172.

Fastener 3228 is a U-shaped member including a pair of substantially rectangular parallelepiped fitted portions 3228a and 3228b that form the end portions of fastener 3228, and curved portion 3228c that is curved into a semicircular shape and that couples fitted portions 3228a and 3228b to each other. Curved portion 3228c of fastener 3228 is engaged with an annular constricted portion 3235, described later, of male screw 3230. The pair of fitted portions 3228a and 3228b of fastener 3228 are fitted respectively to through-holes 3224a and 3224b of cover portion 3224. Male screw 3230 is rotatably fixed to sliding member 3220 with the aid of fastener 3228. In a state in which fixed member 3210 and sliding member 3220 are not coupled (namely, in a state in which male screw 3230 and female screw 3240 are not meshed), fastener 3228 can be easily removed from sliding member 3220.

Male screw 3230 includes engagement portion 3232 capable of engaging with screwdriver 3400, a pair of flange portions 3234 and 3236 arranged to face each other, annular constricted portion 3235 arranged between flange portions 3234 and 3236, and threaded portion 3238 capable of meshing with female screw 3240. Engagement portion 3232, flange portion 3234, constricted portion 3235, flange portion 3236, and threaded portion 3238 are arranged in order mentioned from one end of male screw 3230 to the other end.

Engagement portion 3232 is arranged at a front end of male screw 3230. A shape of engagement portion 3232 is just required to make engagement portion 3232 engageable with screwdriver 3400 and to enable male screw 3230 to be rotated when screwdriver 3400 engaged with engagement portion 3232 is rotated. For example, engagement portion 3232 is in the form of a hexagonal hole, and male screw 3230 can be rotated by inserting a tip end of screwdriver 3400 in the form of a hexagonal prism (namely, a hexagon spanner) that can be fitted to the hexagonal hole.

The pair of flange portions 3234 and 3236 are each formed with the radius of its outer circumference being greater than that of an inner circumference of the curved portion 3228c of fastener 3228, and constricted portion 3235 is formed with the radius of its outer circumference being smaller than that of the inner circumference of the curved portion 3228c of fastener 3228. As a result, movement of male screw 3230 in the front-rear direction is restricted to be held within a spacing between the pair of flange portions 3234 and 3236.

Threaded portion 3238 is arranged on a rear end side of male screw 3230. Threaded portion 3238 is just required to have a thread shape capable of meshing with female screw 3240. A length of threaded portion 3238 is preferably greater than or equal to a half length of male screw 3230 from the viewpoint of increasing an amount by which sliding member 3220 can be advanced relative to fixed member 3210 and increasing an amount by which lower-jaw oral appliance body portion 1112 can be advanced relative to upper-jaw oral appliance body portion 1111.

With positioning member 3160 in this embodiment, the position of lower-jaw oral appliance body portion 1112 relative to the position of upper-j aw oral appliance body portion 1111 can be adjusted by meshing male screw 3230 held on sliding member 3220 with female screw 3240 held on fixed member 3210, and by rotating male screw 3230 to adjust an amount of meshing between male screw 3230 and female screw 3240. For example, by moving lower-jaw oral appliance body portion 1112 forward relative to upper-jaw oral appliance body portion 1111 through a distance in accordance with the jaw shape of the user, the rearward displacement of the lower jaw is restricted, and the airway of the user wearing oral appliance 3100 can be made less likely to narrow.

Fixed member 3210, sliding member 3220, and male screw 3230 can be each made of, for example, a metal material such as titanium, an alloy containing titanium, iron (such as steel containing stainless), gold, silver, platinum, cobalt, chromium, or any alloy of those metals, or a hard resin material (such as polycarbonate resin) with the flexural modulus of higher than or equal to 1000 MPa and lower than or equal to 3000 MPa, that flexural modulus being measured in conformity with JIS T6501. Corrosion resistance of the metal material may be increased with a chemically formed coating, for example.

### (Method of Manufacturing Oral Appliance According to Embodiment 3)

A method of manufacturing oral appliance 3100 according to this embodiment is not limited to a particular one. For example, oral appliance body portions 1110 and support portions 3170 may be fabricated by preparing sheets containing the thermoplastic resin composition and sheets containing the high elastic material and the low elastic material, and by molding the sheets with heat molding (for example, suction molding, pressure molding, or suction and heat molding). Alternatively, the above-mentioned components may be formed by pouring the thermoplastic resin composition, the high elastic material, etc. into molds, or may be fabricated by laser processing, for example. In this embodiment, the above-mentioned components are particularly preferably fabricated by milling or 3D printing. Those methods enable oral appliance body portions 1110 and support portions 3170 to be fabricated with high accuracy. Furthermore, with the milling or the 3D printing, the machining or the processing can be performed even when a material with high transparency to visible light is used for oral appliance body portions 1110 and so on. Oral appliance body portions 1110 and support portions 3170 may be fabricated by the same method or different methods. The method(s) of fabricating oral appliance body portions 1110 and support portions 3170 may be selected as in Embodiment 1.

A method of fabricating each of the components of positioning member 3160 is selected as appropriate depending on the material, and each component can be fabricated by a known metal machining method or a known resin processing method. For example, female screw 3240 may be molded integrally with the other portions of fixed member 3210 (such as cover portion 3214 and mounting portion 3216) by using the same material. Alternatively, female screw 3240, cover portion 3214, mounting portion 3216, and so on m may be fabricated separately, and those components may be joined to each other by a known method.

A method of supporting positioning member 3160 on support portions 3170 is selected as appropriate in conformity with the method of fabricating support portions 3170. Upper support portion 3171 and lower support portion 3172 may be fabricated in advance, and mounting portions 3216 and 3226 of positioning member 3160 may be fixed, res respectively, to surfaces of upper support portion 3171 and lower support portion 3172 with metal fittings, for example. Alternatively, mounting portions 3216 and 3226 may be buried in support portions 3170 by performing 3D printing or heat molding, for example, in a state in which mounting portions 3216 and 3226 of positioning member 3160 are arranged at predetermined positions.

### (Others)

Although any of Embodiments 1 to 3 described above includes the upper support portion covering part of the upper-jaw oral appliance body portion and the lower support portion covering part of the lower-jaw oral appliance body portion, the oral appliance may include only one of the upper support portion and the lower support portion.

### Examples

Oral appliances fabricated in the following Examples and Comparative Example were evaluated on the wearing feel, damage, and deformation in accordance with the criteria described later.

### [Example 1]

Nylon 12 (product name: Lucitone, made by DENTSPLY-Sirona K.K., flexural modulus: 1330 MPa, and density: 1.02 kg/m³) was prepared as the material of the support portions. On the other hand, PMMA (polymethyl methacrylate, flexural modulus: 1731 MPa) was prepared as the material of the oral appliance body portions. Then, oral appliance 1100 illustrated in FIG. 1 was fabricated. The wearing feel of the fabricated oral appliance was good, and neither damage nor deformation was not visually found on the oral appliance taken off after the wearing.

### [Examples 2 and 3]

As listed in Table 1, oral appliances were fabricated in the same manner as in Example 1 except for using, as the material of the support portions, polycarbonate resin (product name: Reining resin N. made by Toushinyoukou Co., Ltd., flexural modulus: 2004 MPa, and density: 1.2 kg/m³) or polyester resin (product name: Estheshot, made by Nissin Dental Products Inc., flexural modulus: 1490 MPa, and density: 1.2 kg/m³). The wearing feel of each of the fabricated oral appliances was good, and the oral appliances were less susceptible to damage and deformation.

### [Example 4]

An oral appliance was fabricated in the same manner as in Example 1 except for using, as the material of the support portions, nickel cobalt metal (product name: Wironit. made by Nissin Dental Products Inc., flexural modulus: 221,000 MPa, and density: 8.2 kg/m³). The fabricated oral appliance was less susceptible to damage and deformation, but the wearing feel deteriorated a little.

### [Example 5]

An oral appliance was fabricated in the same manner as in Example 1 except for using, as the material of the support portions, ethylene-vinyl copolymer (flexural modulus: 100 MPa and density: 1.0 kg/m³). The wearing feel of the fabricated oral appliance was good, but damage and deformation occurred in part of the oral appliance.

### [Comparative Example 1]

An oral appliance was integrally fabricated using PMMA (polymethyl methacrylate, flexural modulus: 1731 MPa) without distinguishing support portions and oral appliance body portions. The wearing feel of the fabricated oral appliance was not good.

**[Table 1]**

| | | Example 1 | Example 2 | Example 3 | Example 4 | Example 5 | Comparative Example 1 |
|---|---|---|---|---|---|---|---|
| Support Portions | Type | Nylon12 | Polycarbonate Resin | Polyester | Nickel Cobalt | EVA | PMMA |
| | Flexural Modulus A (MPa) | 1330 | 2004 | 1490 | 221000 | 100 | 1731 |
| | Density (kg/m³) | 1.02 | 1.2 | 1.2 | 8.2 | 1.0 | 0.95 |
| Oral Appliance | Type | PMMA | PMMA | PMMA | PMMA | PMMA | PMMA |

| Body Portions | Flexural Modulus B (MPa) | 1731 | 1731 | 1731 | 1731 | 1731 | 1731 |
|---|---|---|---|---|---|---|---|
| Difference in Flexural Modulus | B-A (MPa) | -401 | 273 | -241 | 219269 | -1631 | 0 |
| Evaluation | Presence of Damage | A | A | A | A | B | A |
| | Wearing Feel | A | A | A | B | A | D |

In the above Table, the presence of damage was evaluated in accordance with the following criteria. Grades A and B fall in the range where there is no problem in practical use.
A: the oral appliance is not damaged when put on and fitted to a toot row model
B: the oral appliance is partly damaged (flaking is found in parts of the support portions and the oral appliance body portions) when fitted to the tooth row model
C: the oral appliance is damaged when fitted to the tooth row model
D: the oral appliance is damaged before being fitted to the tooth row model

The wearing feel was evaluated in accordance with the following criteria. Grades A and B fall in the range where there is no problem in practical use.
A: the tooth row model does not drop when the oral appliance is held after fitting the oral appliance to the tooth row model
B: the tooth row model is partly damaged when the oral appliance is fitted to the tooth row model
C: the tooth row model is damaged when the oral appliance is fitted to the tooth row model
D: the oral appliance cannot be fitted to the tooth row model or/and the tooth row model drops when, after fitting the oral appliance to the tooth row model, the oral appliance is held while it is kept fitted to the tooth row model

As seen from above Table 1, the oral appliance fabricated using one material could not be fitted to the tooth row model, and the wearing feel was not good (Comparative Example 1). By contrast, the wearing feel of the oral appliances each fabricated using materials different in level of flexural modulus for the support portions and the oral appliance body portions was good, and the oral appliances were less susceptible to damage (Examples 1 to 5). Particularly, when an absolute value of the difference in flexural modulus between the support portions and the oral appliance body portions was higher than or equal to 100 MPa and lower than or equal to 1600 MPa (Examples 1 to 3), no damage occurred, and the wearing feel was very good.

This application claims priority based on Japanese Patent Application No. 2019-170696 filed September 19, 2019. The contents disclosed in Specification of the cited Japanese Patent Application and the attached drawings are all incorporated in Description of this application by reference.

### Industrial Applicability

According to the oral appliance of the present invention, since the rearward displacement of the lower jaw of the user can be restricted, the oral appliance is very effective in prevention and treatment of the sleep apnea syndrome. Furthermore, the oral appliance of the present invention gives more comfortable wearing feel than the related art and is less susceptible to damage and deformation. Hence the oral appliance of the present invention can be applied to not only prevention and treatment of the sleep apnea syndrome, but also prevention and treatment of temporomandibular disorders, suppression of bruxism, and so on.

### Reference Signs List

1100, 2100, 3100 Oral appliance
1110 Oral appliance body portion
1111 Upper-jaw oral appliance body portion
1111a, 1112a Tooth row mold portion
1112 Lower-jaw oral appliance body portion
1130 Upper holding portion
1132 Upper shaft
1134 Upper flange portion
1140 Lower holding portion
1142 Lower shaft
1144 Lower flange portion
1150 Coupling portion
1152 Outer
1154 Inner
1160, 3160 Positioning member
1170, 3170 Support portion
1171, 2171, 3171 Upper support portion
1172, 2172, 3172 Lower support portion
2130 Stopper
2132 Front surface of stopper
2136 Inclined portion of stopper
2140 Wing
2142 Rear surface of wing
2146 Inclined portion of wing
2150 Adjustment portion
2152 Second screw rod
2154 First screw rod
2156 Turnbuckle
2160 Movable wing
3210 Fixed member
3212 Groove portion of fixed member
3214 Cover portion of fixed member
3216, 3226 Mounting portion
3216a, 3226a Through-hole
3220 Sliding member
3222 Groove portion of sliding member
3224 Cover portion of sliding member
3224a, 3224b Through-hole
3228 Fastener
3228a, 3228b Fitted portion
3228c Curved portion
3230 Male screw
3232 Engagement portion
3234, 3236 Flange portion
3235 Constricted portion
3238 Threaded portion
3240 Female screw
3400 Screwdriver

## Claims

1. An oral appliance, comprising:
a pair of oral appliance body portions constituted by an upper-jaw oral appliance body portion fitted to an upper jaw and a lower-jaw oral appliance body portion fitted to a lower jaw;
a positioning member that restricts rearward displacement of the lower-jaw oral appliance body portion; and
a support portion that is disposed between the positioning member and at least one of the oral .appliance body portions to cover part of a surface of at least one of the upper-jaw oral appliance body portion and the lower-jaw oral appliance body portion, the surface being opposite to another surface fitted to a tooth row, and that supports the positioning member,
wherein the support portion comprises a material with a different flexural modulus from the oral appliance body portions.

2. The oral appliance according to claim 1, wherein:
an absolute value of difference between the flexural modulus of the oral appliance body portions and the flexural modulus of the support portion is greater than or equal to 100 MPa and smaller than or equal to 1600 MPa.

3. The oral appliance according to claim 1 or 2, wherein:
a density of the support portion is smaller than 1.5 kg/m³.

4. The oral appliance according to any one of claims 1 to 3, wherein:
the flexural modulus of the oral appliance body portions is 50 to 3000 MPa.

5. The oral appliance according to any one of claims 1 to 4, wherein:
the oral appliance body portions comprise polyester resin, polycarbonate resin, polyamide resin, or (meth)acrylic resin.

6. The oral appliance according to any one of claims 1 to 5, wherein:
the support portion comprises metal or (meth)acrylic resin.

7. The oral appliance according to any one of claims 1 to 6, comprising:
a pair of left and right support portions.
